(19) Europäisches Patentamt
European Patent Office
Office européen des brevets

(11) Publication number: **0 353 211 A1**

(12) # EUROPEAN PATENT APPLICATION

(21) Application number: **89850205.9**

(22) Date of filing: **20.06.89**

(51) Int. Cl.5: **C 07 C 323/41**
C 07 C 317/28, C 07 K 5/00,
A 61 K 31/165, A 61 K 37/64

(30) Priority: **28.06.88 SE 8802428**

(43) Date of publication of application:
**31.01.90 Bulletin 90/05**

(84) Designated Contracting States:
**AT BE CH DE ES FR GB GR IT LI LU NL SE**

(71) Applicant: **Aktiebolaget Hässle**
**Kärragatan 5**
**S-431 83 Mölndal (SE)**

(72) Inventor: **Karlsson, Jan Olle**
**Gundefjällsgatan 377**
**S-431 45 Mölndal (SE)**

**Sohtell, Erik Morgan Herman**
**Lindomebyvägen 27**
**S-437 00 Lindome (SE)**

**Westerlund, Rolf Christer**
**Alegardsgatan 18**
**S-431 50 Mölndal (SE)**

(74) Representative: **Näsman, Rune B. G. et al**
**AB Astra Patent and Trade Mark Department**
**S-151 85 Södertälje (SE)**

Claims for the following Contracting States: ES + GR.

(54) **New compounds.**

(57) Compounds having a renin inhibitory effect and having the formula

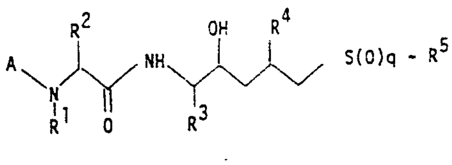

I

where

A = 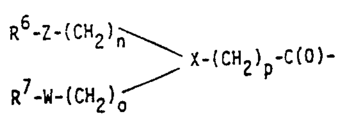

$R^1$ = H; lower alkyl (1-3 carbons)
$R^2$ = straight or branched lower alkyl or alkenyl (1-6 carbons), optionally substituted with lower alkoxy, cyano, fluoro, lower thioalkyl; cycloalkyl (3-7 carbons); aryl (6-10 carbons); cycloalkylalkyl (4-9 carbons); arylalkyl (7-11 carbons)
$R^3$ = straight or branched lower alkyl (3-6 carbons); cycloalkylalkyl (4-11 carbons); arylalkyl (7-11 carbons)
$R^4$ = H; straight or branched lower alkyl (1-5 carbons)
$R^5$ = straight or branched lower alkyl (1-5 carbons); cycloalkyl (3-7 carbons); aryl (6-10 carbons); arylalkyl (7-11 carbons); cycloalkylalkyl (4-8 carbons)
q = 0-2
o = 0-2
p = 0-2
n = 0-2
X = CH; N
Z = O; CH($R^8$); absent
W = O; CH($R^8$); absent
$R^6$ = straight or branched lower alkyl (1-6 carbons); cycloalkyl (3-7 carbons); aryl (6-10 carbons) either as such or substituted by 1-3 substituents selected from: halogen (e.g. F,Cl), lower alkoxy (1-3 carbons e.g. methoxy), nitro, hydroxy, lower alkyl (1-3 carbons e.g. methyl), cyano
$R^7$ = as defined for $R^6$
$R^8$ = lower alkyl (1-3 carbons).
Processes for preparation and pharmaceutical preparations for such compounds and methods for treatment of heart disorders with such compounds are further disclosed.

EP 0 353 211 A1

## Description

## New compounds

Background

Renin is a natural proteolytic enzyme, which is released into the blood from the kidney. Its only known function is cleave circulating angiotensinogen into angiotensin I, which is a decapeptide of the following structure:

Asp-Arg-Val-Tyr-Ile-His-Pro-Phe-His-Leu

This in turn is further cleaved by angiotensin converting enzyme (ACE), resulting in the formation of the octapeptide angiotensin II:

Asp-Arg-Val-Tyr-Ile-His-Pro-Phe

This peptide is one of the most potent blood-pressure elevating agents known. It is biological activity in this respect is partly due to a vasoregulating effect and partly on an aldosterone mediated antidiuretic and antisaliuretic effect.

Efforts to control hypertension by way of the reinangiotensin system has until lately been mainly directed toward inhibition of ACE. Although this principle has proved clinically useful, the non-specificity of ACE has been advocated as a reason for different side-effects. Since renin is highly specific for angiotensinogen, inhibitors of this enzyme could provide advantageous in the combat against different forms of hypertension.

Prior art

The earliest efforts towards making renin inhibitors was mainly directed towards simple substrate analogues. By variations in the amino acid sequence Haber et al succeeded in increasing their potency. Further effectiveness was achieved by replacing the scissile dipeptide unit in the inhibitors with a non-cleavable unit such as statine (Boger et al Nature, 303, 81 (1983) or different isosteres (Szelke et al Nature, 299, 555 (1982). Compounds of this type provided very potent but had short duration of effect. This was attributed to proteolytic instability of the peptides, and thus much of the more recent work in this area has been directed towards making smaller inhibitors with reduced number of potentially scissile peptide bonds. For some recent publications on this subject see Matsuda et al, Chem. Lett. 1041 (1985), Plattner et al, 191th ACS-meeting, New York, (1986), Hanson et al, Biochem. Biophys. Res. Comm. 132, 155 (1985) and Toda et al, Eur. J. Pharm. 129, 393 (1986) Recent patent applications relating to shorter inhibitors include EP 186977 (Sankyo), EP 190891 (Kissei), EP 172346 (Abbott), EP 189203 (Abbott), EP 172347 (Abbott) and EP 181110 (Kissei).

Definitions and abbreviations

Definitions

The following definitions apply both to the description of the invention and the claims unless otherwise specified.

1. The term "amino acid" includes amino acids of both natural and unnatural origin.
2. All amino acids can be of either L- or D-configuration but are preferably of the L-configuration unless otherwise stated.
3. All asymmetric centers may be of either R or S configuration unless otherwise stated.
4. Reference to carboxylic acids and amino acids should be read as the corresponding acyl residues unless it is apparent from the context that the free acid is included.
5. The term "aryl" means a carbocyclic or heterocyclic aromatic ring and includes phenyl, thienyl, pyridyl and naphtyl. The number of carbon atoms given for aryl includes ring heteroatoms when occurring.

Abbreviations

Dba = Dibenzylacetic acid
Agl = Allylglycine
Cha = Cyclohexylalanine
Ala = Alanine
Ape = 2-Aminopentanoic acid (= Nva)
Dnma = Di(1-naphtylmethyl)acetic acid
His = Histidine
Dtma = Di(2-thienylmethyl)acetic acid
Cpg = Cyclopentylglycine
Cpra = Cyclopropylalanine
Bnma = Benzyl(1-naphtylmethyl)acetic acid
Cya = Cyanoalanine
Dpa = Diphenylacetic acid
Dpp = 3,3-Diphenylpropanoic acid
Dcma = Di(cyclohexylmethyl)acetic acid

Tal = 2-Thienylalanine
Phg = Phenylglycine
Val = Valine
Dbc = Dibenzylcarbamic acid
Fape = 5,5,5-trifluoro-2-aminopentanoic acid
Bcma = Benzyl(cyclohexylmethyl)acetic acid
Dba(2,3,2′,3′-Cl) = Di(2,3-dichlorobenzyl)acetic acid
Dba(2,3,2′,3′-Me) = Di(2,3-dimethylbenzyl)acetic acid
Dba(4,4′-OH) = Di(4-hydroxybenzyl)acetic acid
Dba(4-OH) = Benzyl(4-hydroxybenzyl)acetic acid
Dba(4,4′-OMe) = Di(4-methoxybenzyl)acetic acid
Dba(4,4′-$NO_2$) = Di(4-nitrobenzyl)acetic acid
Dba(4,4′-Cl) = Di(4-chlorobenzyl)acetic acid
Dpea = Di(phenylethyl)acetic acid
Leu = Leucine
Gly = Glycine
Bpma = Benzyl(4-pyridylmethyl)acetic acid
Ips = O-Isopropylserine
[OH] = CH(OH)-$CH_2$
[R] = $CH_2$
Boc = t-Butoxycarbonyl

<u>Disclosure of the invention</u>

This invention relates to new types of short renin inhibitors, their synthesis, pharmaceutical composition containing the compounds as active ingredients and the use of the compounds as drugs for treatment of hypertension, congestive heart failure and other cardiovascular disorders.

Thus it has been found that compounds of the general formula

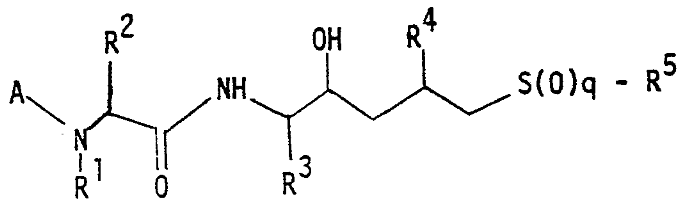

where

A = 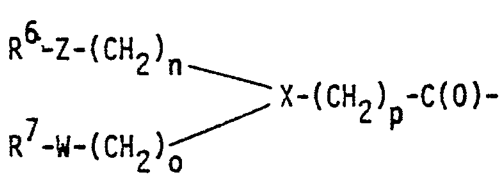

$R^1$ = H; lower alkyl (1-3 carbons)
$R^2$ = straight or branched lower alkyl or alkenyl (1-6 carbons), optionally substituted with lower alkoxy, cyano, fluoro, lower thioalkyl; cycloalkyl (3-7 carbons); aryl (6-10 carbons); cycloalkylalkyl (4-9 carbons); arylalkyl (7-11 carbons)
$R^3$ = straight or branched lower alkyl (3-6 carbons); cycloalkylalkyl (4-11 carbons); arylalkyl (7-11 carbons)
$R^4$ = H; straight or branched lower alkyl (1-5 carbons)
$R^5$ = straight or branched lower alkyl (1-5 carbons); cycloalkyl (3-7 carbons); aryl (6-10 carbons); arylalkyl (7-11 carbons); cycloalkylalkyl (4-8 carbons)
q = 0-2
o = 0-2
p = 0-2
n = 0-2
X = CH; N
Z = O; CH($R^8$); absent
W = O; CH($R^8$); absent
$R^6$ = straight or branched lower alkyl (1-6 carbons); cycloalkyl (3-7 carbons); aryl (6-10 carbons) either as such or substituted by 1-3 substituents selected from: halogen (e.g. F,Cl), lower alkoxy (1-3 carbons e.g. methoxy), nitro, hydroxy, lower alkyl (1-3 carbons e.g. methyl), cyano
$R^7$ = as defined for $R^6$
$R^8$ = lower alkyl (1-3 carbons);
show renin inhibitory effect; however excluding from the scope of this patent application any compound

disclosed in International Patent Application No PCT/SE87/00633, published under No WO 88/05049, and specifically excluding the compound of the formula

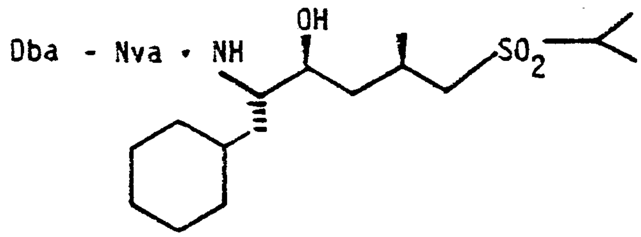

i. e. excluding the compound of formula I above wherein A = Dba (i.e. p = 0, X = CH, n = o = 1, Z = W = absent, $R^6$ = $R^7$ = phenyl), $R^1$ = H, $R^2$ = propyl, $R^3$ = cyclohexylmethyl, $R^4$ = methyl, q = 2 and $R^5$ = $-CH(CH_3)_2$; either as such or in the form of a physiologically acceptable salts and including optical isomers.

The compounds of formula I may be employed alone or in a combination for treating hypertension, congestive heart failure and other cardiovascular disorders, comprising a renin inhibitor according to formula I and one or more cardiovascular agents selected from the group comprising diuretics such as amiloride, bumetanide, chlortalidon, furosemide, gendroflumethiazide, hydrochlorothiazide and spironolactone;
α-adrenergic blocking agents such as prazosin;
β-adrenergic blocking agents such as atenolol, betaxolol, metoprolol, pindolol, propranolol and timolol;
α- and β-adrenergic blocking agents such as labetalol;
CNS-agents such as clonidine and methyldopa;
vasodilators such as hydralazine, isosorbide dinitrate, isosorbide mononitrate and nitroglycerine;
ACE-inhibitors such as captopril, enalapril, lisinopril, and ramipril; and
Ca-antagonists such as amlodipine, diltiazem, felodipine, nifedipine, nitrendipine, and verapamil.

Preferred compounds are

Dba-Agl-Cha-[OH]-Ala-[R]-$SO_2$-$CH(CH_3)_2$
Dnma-Ape-Cha-[OH]-Ala-[R]-$SO_2$-$CH(CH_3)_2$
Dba-Ape-Cha-[OH]-Ala-[R]-$SO_2$-$CH_3$
Dba-Agl-Cha-[OH]-Ala-[R]-$SO_2$-cyclohexyl
Dba-Ape-Cha-[OH]-Ala-[R]-$SO_2$-phenyl
Dnma-Agl-Cha-[OH]-Ala-[R]-$SO_2$-$CH_2$-phenyl
Dba(2,3,2',3'-Cl)-Ape-Cha-[OH]-Ala-[R]-$SO_2$-cyclohexyl
Dba(4,4'-OH)-Ape-Cha-[OH]-Ala-[R]-$SO_2$-$CH(CH_3)_2$
Dba(4-OH)-Ape-Cha-[OH]-Ala-[R]-$SO_2$-$CH(CH_3)_2$
Dba(4,4'-Cl)-Ape-Cha-[OH]-Ala-[R]-$SO_2$-cyclohexyl
Dtma-Cpg-Cha-[OH]-Ala-[R]-$SO_2$-$CH(CH_3)_2$
Dba-Cpg-Cha-[OH]-Ala-[R]-$SO_2$-$CH(CH_3)_2$
Bnma-Val-Cha-[OH]-Ala-[R]-$SO_2$-$CH(CH_3)_2$
Dnma-Phg-Cha-[OH]-Ala-[R]-$SO_2$-$CH(CH_3)_2$
Dnma-Agl-Leu-[OH]-Ala-[R]-$SO_2$-$CH(CH_3)_2$
Dba-Ape-Cha-[OH]-Gly-[R]-$SO_2$-$CH(CH_3)_2$
Dnma-Agl-Cha-[OH]-Gly-[R]-$SO_2$-$CH(CH_3)_2$
Dba-Agl-Cha-[OH]-Val-[R]-$SO_2$-$CH(CH_3)_2$
Dnma-Ape-Cha-[OH]-Val-[R]-$SO_2$-$CH(CH_3)_2$
Bpma-Cpra-Cha-[OH]-Ala-[R]-$SO_2$-$CH(CH_3)_2$
Dba-Cpra-Cha-[OH]-Ala-[R]-$SO_2$-$CH(CH_3)_2$
Bpma-Agl-Cha-[OH]-Ala-[R]-$SO_2$-$CH(CH_3)_2$

A further objective of the invention is the mode of preparation of the compounds of the invention. Thus the synthesis of

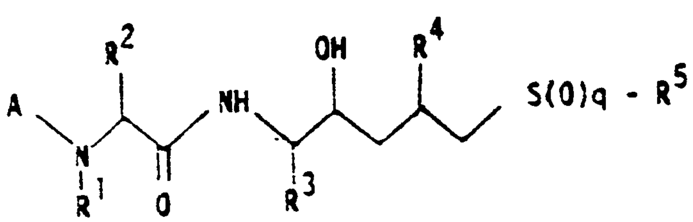

I

is essentially achieved by first assembling the

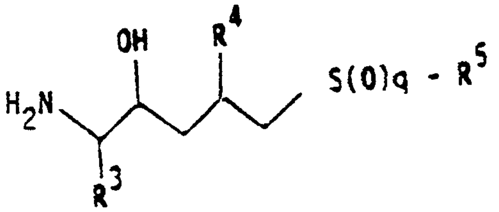

portion henceforth referred to as the isostere, followed by one or two coupling reactions, optionally followed or intervened by some further manipulations of the isostere part, thus, leading to structures of formula I.

Thus the invention further relates to a process for preparation of compounds according to formula I, in which process an isostere of the formula

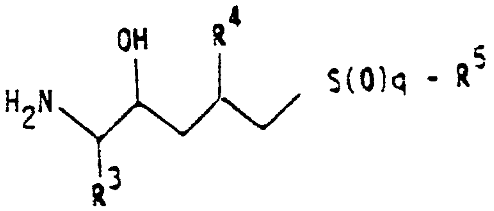

wherein $R^3$, $R^4$, $R^5$ and q are as defined with formula I, is coupled to appropriate amino acids by standard peptide synthesis techniques, and in those cases where the reactions result in a mixture of diastereomers these are optionally separated by standard chromatographic or recrystallization techniques, and if desired an optical isomer is isolated.

Preferably the isostere is reacted at the N-terminal with an amino acid derivative of the formula

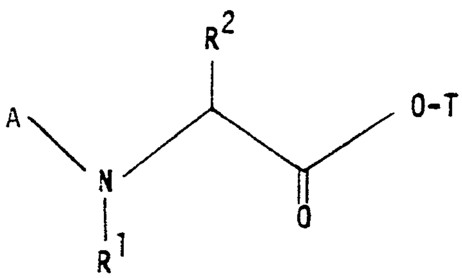

wherein $R^1$, $R^2$ and A are as defined with formula I and T is an activating group standardly used in peptide synthesis and preferably selected from -C(O)$R^{10}$ or -C(O)O$R^{10}$, wherein $R^{10}$ is a straight or branched lower alkyl, N-benzotriazolyl, N-succinimidyl, nitrophenyl or azido in an inert solvent such as methylene chloride, chloroform, ethyl acetate, toluene, dimethoxyethane, DMF or THF at a temperature of -50 to 100°C, to the formation or a compound of formula I, either directly or after deprotection, or with an amino acid derivative of the formula

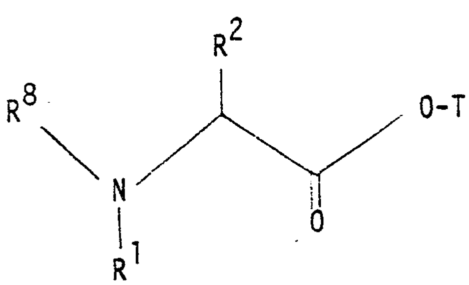

wherein $R^1$ and $R^2$ are as defined with formula I, $R^8$ is a protecting group and T is as defined above, in an inert solvent such as methylene chloride, chloroform, ethyl acetate, toluene, dimethoxyethane, DMF or THF at a temperature of -50 to 100°C, then removing the protecting group $R^8$, and introducing a group A - T wherein A is as defined with formula I and T is as defined above in an inert solvent such as methylene chloride, chloroform, ethyl acetate, toluene, dimethoxyethane, DMF or THF at a temperature of -50 to 100°C, to the formation of a compound of formula I, either directly or after deprotection.

I. Synthesis of isosteres

The moieties

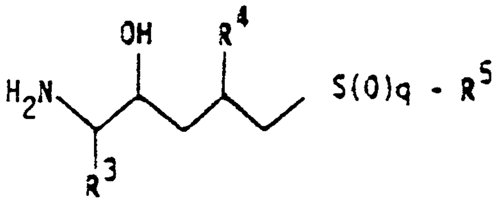

hereinafter referred to as the isosteres are prepared according to the following methods

Step 1

Method A

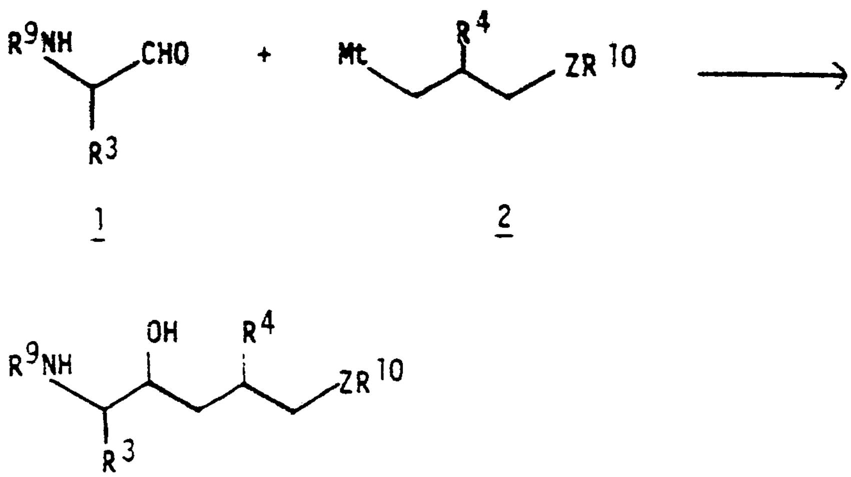

1

2

3

where an organometallic reagent 2 attacks an amino aldehyde 1 in a protected form to give compound 3 (Z = O or S)

Method B

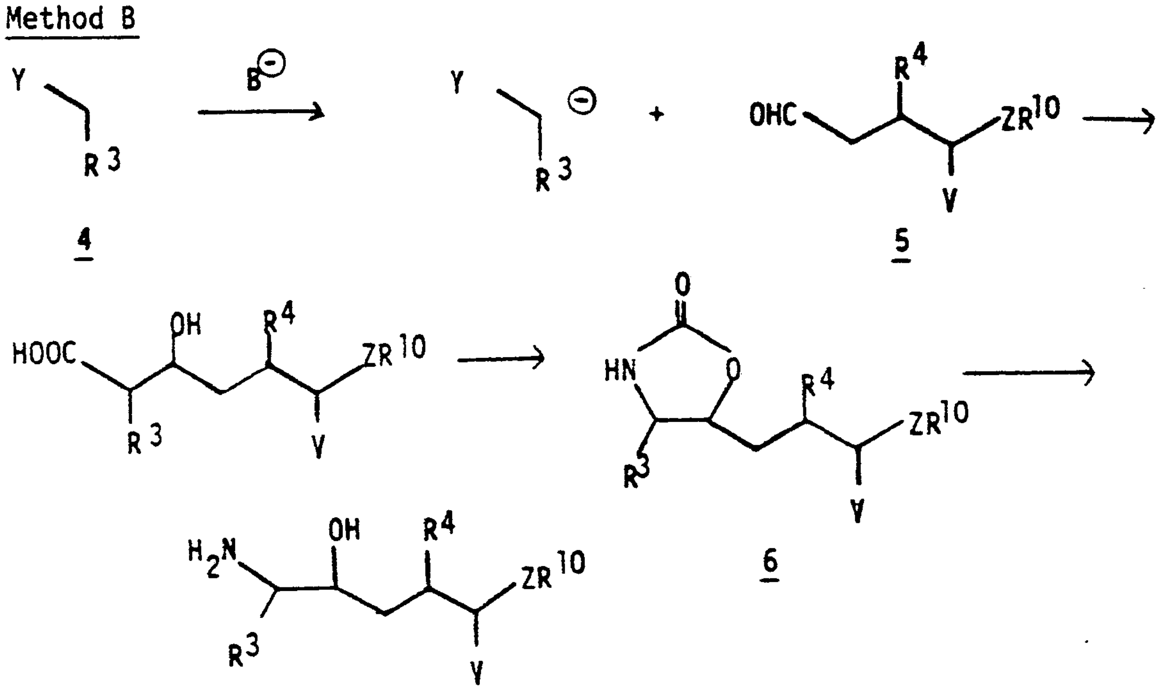

4 5 6 3a

where an appropriate carboxylic acid derivative 4 and an aliphatic aldehyde 5 react in an aldol reaction producing, after a Curtius rearrangement of the liberated carboxylic acid, an oxazolidinone 6 which can be cleaved by alkaline hydrolysis to give the desired amino alcohol 3a (Z = O, S)

Step 2

Conversion of Z = O into Z = S and of Z = S into Z = SO or $SO_2$

The oxygen is introduced as an ether or ester. At some step in the synthesis cleavage of the ethers and reduction of the esters respectively yield the alcohol 7

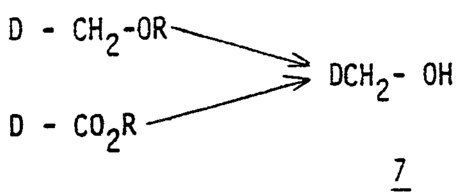

7

The oxygen in 7 is replaced by sulfur by first transforming the primary alcohol into a leaving group, thus 8 is formed. The sulfur is then introduced by treating 8 with a thiolate. The resulting thioether 9 can be oxidized to sulfoxide 10 or sulfone 11 prior to or after the coupling of the acids.

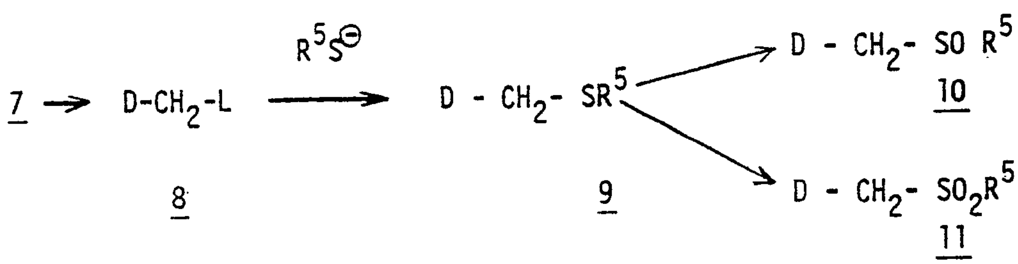

10 11 8 9

Certain precautions have to be taken when 8 is synthesized:

In these hydroxy isosteres the secondary hydroxy group has to be protected, e.g. in the form of an oxazolidinone 8a.

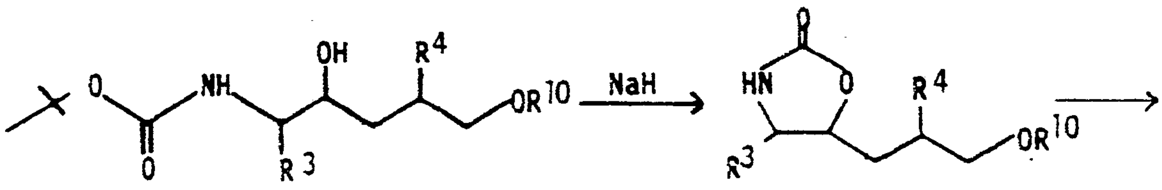


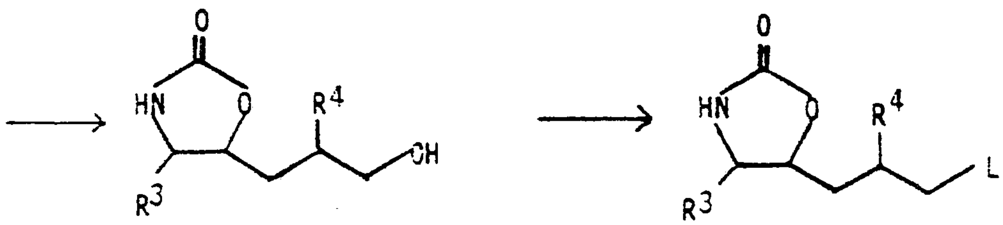

8a

After the necessary transformations the oxazolidinone ring can then be cleaved to give the free amino alcohol 12.

8a

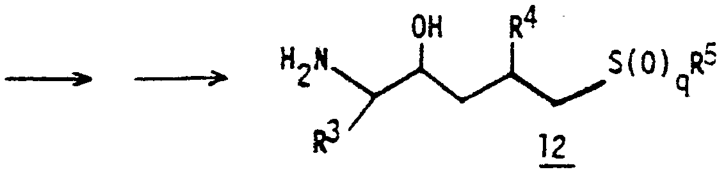

12

II. Coupling of isosteres to amino acids and N-terminal groups

This is done by use of stadard peptide and amide synthesis techniques such as coupling the isosteres with the hydroxybenzotriazole and hydroxysuccinimide esters of the appropriate acids either in two separate steps

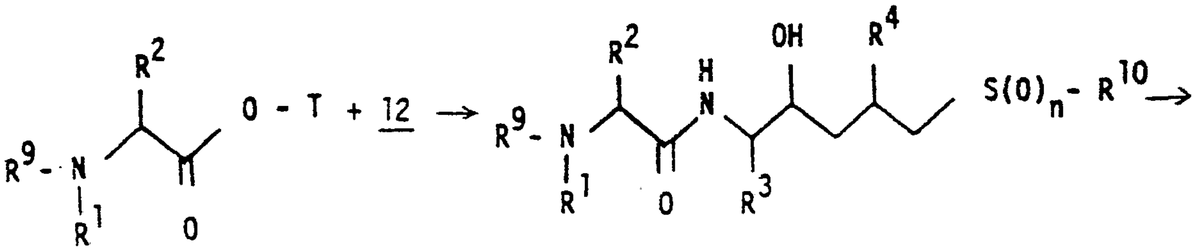


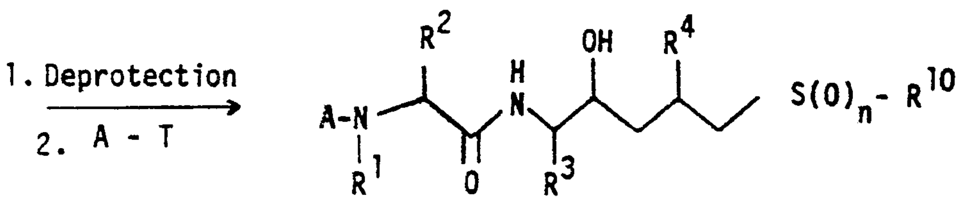


or in one step

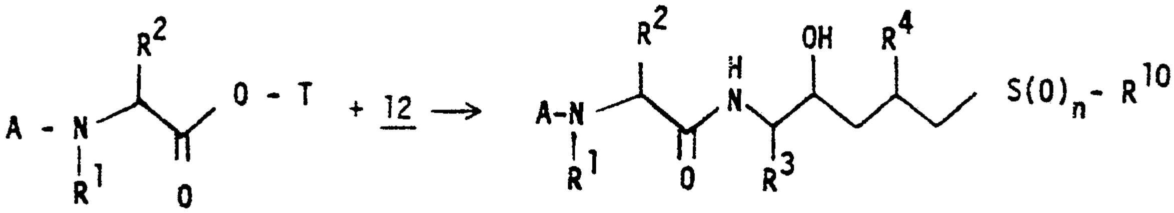

III. Separation of diastereomers

In those cases where the reactions result in a mixture of diastereomers these can be separated by standard chromatographic or recrystallization techniques.

IV. Preparation of starting materials

$\alpha$, $\alpha$-disubstituted acids defined by A are prepared by standard alkylation of malonic ester derivatives, followed by hydrolysis and decarboxylation. In the case where the two $\alpha$-substituents are different the resulting compounds can be used as racemates. Dpa is commercially available, Dmca is obtained by catalytic hydrogenation of Dba and Dpea is obtained by hydrolysis of the corresponding nitrile prepared from 3-bromo-1,5-diphenylpentane.

The amino acids used are either commercially available, or are prepared according to published methods.

The symbols used in the formulas above in paragraphs I-IV retain their meaning if previously defined and otherwise refer to the following:

Z = O; $S(O)_n$

Mt = a metal

R = lower alkyl

$R^9$ = N-protecting group

$R^3$ = as described above

$R^4$ = as described above

$R^{10}$ = O-protecting group if Z = O and $R^5$ if Z = $S(O)_n$

Y = Carboxylic groupe or equivalent

$R^5$ = as described above

V = $H_2$; =0

L = a leaving group

D = 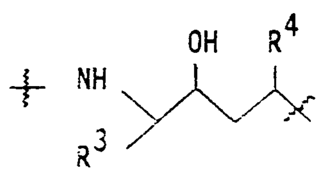

with or without any amino acid residues attached to the nitrogen. A = as described above

T = an activating group

A further objective of the invention is a pharmaceutical composition containing the compounds of the invention. Thus they can be formulated for use in the reduction of blood pressure in compositions such as tablets, capsules or elixirs for oral or rectal administration or in sterile solutions or suspensions for parenteral or nasal administration. About 0,001 to 500 mg of a compound is then compounded with a physiologically acceptable vehicle, carrier, excipient, binder, preservative, stabilizer, flavour etc. in a unit dosage form as called for by accepted pharmaceutical practice. The amount of active compound in these preparations is such that a suitable dosage in the range indicated is obtained. Variations and changes which are obvious to one skilled in the art of formulations are intended to be within the scope of the invention.

A further objective of the invention is the use of the compounds as drugs for treatment of hypertension, congestive heart failure or other cardiovascular disorders. Thus for example by administration of a composition containing one of the compounds of the invention as active ingredient to a patient suffering from hypertension a reduction in blood pressure is obtained. The substance is preferably administered orally, but parenteral, rectal and nasal routes can also be used. The dosage is preferably 0.001 to 500 mg of active ingredient and is preferably administered 1-3 times a day.

Best mode of carrying out the invention

The following examples illustrates the principles of the invention in more detail.

Example 1

Preparation of: Dba-Agl-Cha-[OH]-Ala-[R]-$SO_2$-$CH(CH_3)_2$

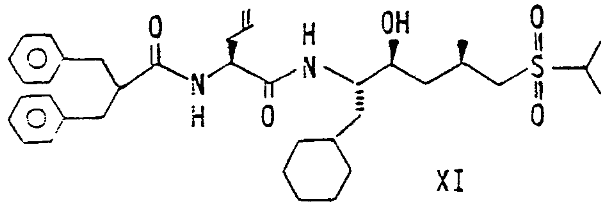

XI

a) Preparation of

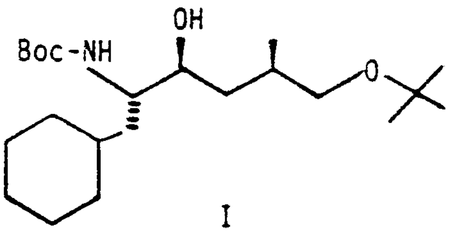

I

The intermediate II was prepared according to N. Cohen et al., J. Org. Chem. 41, 3505 (1976) and intermediate III was prepared according to J. Boger et al., J. Med. Chem. 28, 1779 (1985).

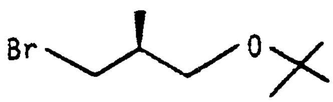

II

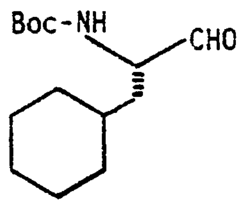

III

To a stirred mixture of 1.4 g (0.057 mol) of powdered magnesium in 14 ml of anhydrous THF were added a crystal of iodine and a few drops of a solution of 9.9 g (0.048 mol) of II in 20 ml of dry THF. The mixture was brought to reflux and after the reaction had started the remainder of the solution of II was added at such a rate that reflux was maintained. After the addition was complete, the reaction mixture was stirred at reflux for an additional 1 h, then cooled to 0-5°C whereupon a solution of 4.9 g (0.019 mol) of III in 35 ml of dry THF was added dropwise while keeping the temperature below 10°C. The reaction mixture was stirred for 3 h at room temperature and was then poured onto 30 ml of saturated ammonium chloride solution and 30 ml of water. 120 ml of ether was added and the mixture was stirred vigorously. The aqueous layer was extracted with 2x40 ml of ether. The combined organic layers were dried over sodium sulfate and the ether was evaporated. The crude product was chromatographed on silica gel and eluted with petroleum ether/ethyl acetate 5:1. Yield: 3.4 g (46%) of I and 0.65 g (9%) of the diastereomeric Ib.

b) Preparation of

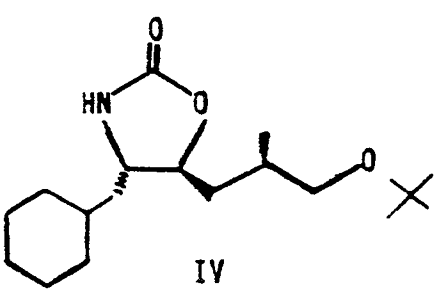

IV

To a solution of 1.5 g (3.9 mmol) of I in 15 ml of dimethylformamide was added 0.24 g (10 mmol) of sodium hydride (55% dispersion in mineral oil). The mixture was stirred for 5 h at room temperature and then poured

onto 7 ml of saturated ammonium chloride solution and 7 ml of water. The mixture was extracted with 3x30 ml of methylene chloride. The combined organic layers were dried over sodium sulfate and evaporated to give 1.1 g (91%) of IV which was used without further purification in the next step.

c) Preparation of

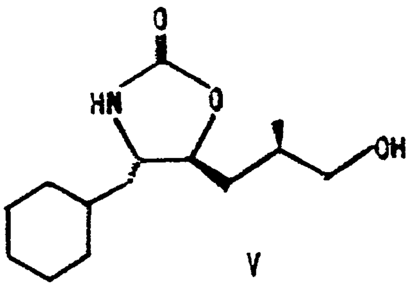

V

A solution of 1.1 g of IV in 6 ml of 4 M HCl and 6 ml of dioxane was heated on a 90°C water bath for 1 h. The reaction mixture was evaporated and then chromatographed on silica gel with ethyl acetate. Yield: 0.50 (56%) of V. Alternatively, IV can be treated with 2 eq. of trimethylsilyl iodide in methylene chloride for 30 min. The organic phase is then washed with $Na_2S_2O_3$(aq), water and dried ($MgSO_4$) giving the product.

d) Preparation of

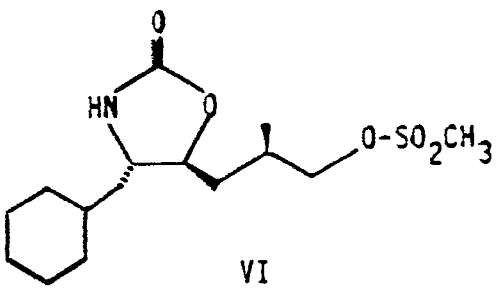

VI

A solution of 0.50 g (1.9 mmol) of V and 0.23 g (2.2 mmol) of triethylamine in 5 ml of methylene chloride was cooled to 0°C. Methanesulfonyl chloride, 0.24 g (2.1 mmol), was added. The reaction mixture was stirred for 2 h and the reaction was monitored by TLC. A 5 ml portion of petroleum ether (40-60°) was added and triethylammonium chloride was filtered off. The solution was evaporated to give 0.75 g (100%) of VI which was used directly in the next step.

e) Preparation of

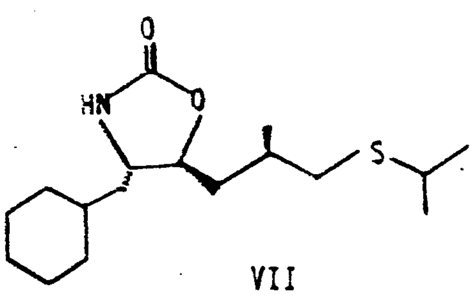

VII

To a solution of 0.75 g (1.9 mmol) of VI in 5 ml of dry THF was added a suspension of sodium isopropyl thiolate - generated from 0.18 g (2.3 mmol) of isopropylthiol and 0.11 g (2.5 mmol) of sodium hydride (55% dispersion in mineral oil) - in 5 ml of dry THF. The reaction mixture was stirred for 2h. The reaction was monitored by TLC. Then 15 ml of methylene chloride and 5 ml of water were added and the mixture was vigorously stirred. The two layers were separated and the aqueous one was extracted with 5 ml of methylene chloride. The combined organic layers were dried over sodium sulfate and then evaporated to give 0.62 g

(100%) of VII which was used directly in the next step.

f) Preparation of

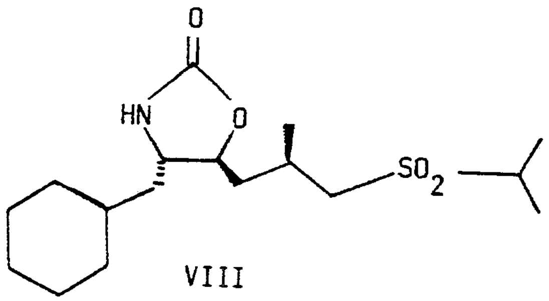

VIII

A solution of 0.50 g (1.6 mmol) of VII and 1.0 g of 55% meta-chloroperbenzoic acid (3.2 mmol) in 20 ml of methylene chloride was stirred for 3 h at room temperature. The mixture was then washed with 50 ml of 1M NaOH, then with water and finally dried over sodium sulfate and evaporated. The crude product was flash chromatographed on silica gel with petroleum ether-ethyl acetate 1/1. Yield: 0.43 g (78%).

g) Preparation of

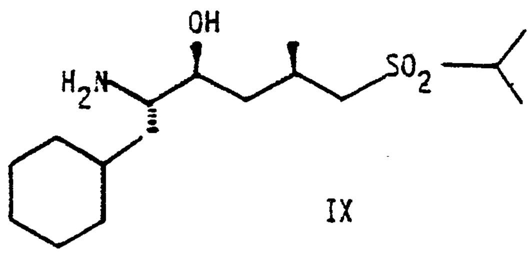

IX

A suspension of 0.43 g (1.2 mmol) of VIII and 0.28 g (5.0 mmol) of potassium hydroxide in 10 ml of ethanol was stirred and heated to 100°C overnight. The reaction mixture was poured into water and extracted with methylene chloride three times. The combined organic fractions were washed with water, dried over sodium sulfate and evaporated. The crude product started to crystallize and was used as such in the ensuing step. Yield: 0.34 g (86%).

h) Preparation of

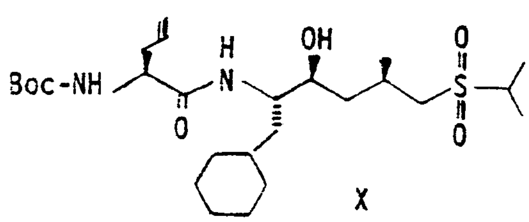

X

To an ice cooled mixture of 0.29 g (1.4 mmol) of Boc-Agl-OH and 0.37 g of (2.7 mmol) of hydroxybenzotriazole in 6 ml of methylene chloride was added 0.66 g (1.6 mmol) of N-cyclohexyl-N′-(2-morpholinoethyl)carbodiimide-metho-p-toluenesulfonate (CME-CDI). After 20 minutes the ice bath was removed and the mixture was then stirred for 3 h. The solution was removed by evaporated. The residue was dissolved in 5 ml of dry dimethylformamide and cooled to 0°C, whereupon a solution of 0.34 g (1.1 mmol) of IX was added and the pH adjusted to 8 by addition of N-methylmorpholine. The ice bath was removed after 30 minutes and the reaction mixture was then stirred overnight. It was then poured into water and extracted three times with ethyl acetate. The combined organic phase was washed twice with 0.5 M HCl, once with saturated $NaHCO_3$ (aq) and twice with water. Drying and evaporation gave the crude product as crystals, which was used without further purification in the next step. Yield 0.41 g (75%).

i) Preparation of

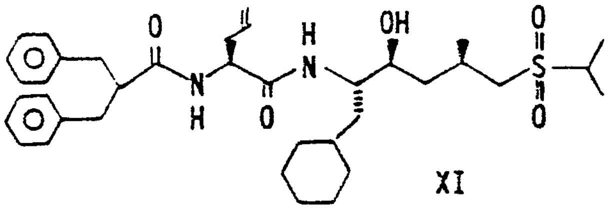

XI

The hydroxybenzotriazole ester of dibenzylacetic acid was prepared (by the same method as described in the previous step) from 0.21 g (087 mmol) of Dba, 0.24 g (1.7 mmol) of hydrobenzotriazole and 0.42 g (0.99 mmol) of CME-CDI in 10 ml of methylene chloride. Compound X, 0.41 g (0.79 mmol), was dissolved in a mixture of 1.8 ml of methylene chloride and 0.60 ml of trifluoroacetic acid. After 1.5 h the mixture was evaporated and redissolved in 5 ml of dry dimethylformamide and added to solution of the hydroxybenzotriazole ester in 5 ml of dry dimethylformamide at 0° C. The pH was adjusted to 8 by addition of N-methyl morpholine and the reaction mixture was then stirred at room temperature for 3 days. The same work-up procedure was used as in the previous step, and the crude produkt was purified by flash chromatography using petroleum ether/ethyl acetate (1/1). Yield.0.31 g (61 %).

Example 2-46 (see tables 1 and 2)

The preparation of these compounds was accomplished in a similar way as described above in example 1.

Example 47

Solution for injection

A solution is prepared from the following ingredients:

| | |
|---|---|
| Renin inhibitor | 1 g |
| Ethanol (99.5 %) | 100 ml |
| Polyethylene glycol 400 | 400 ml |
| Water for inj. up to | 1000 ml |

The active constituent is dissolved in the ethanol/polyethylene glycol mixture. Water is added to final volume, whereafter the solution is filtered through a sterile 0,2 µm filter and aseptically filled into sterile ampoules (5 ml).

Example 48

Solution for injection

A solution is prepared from the following ingredients:

| | |
|---|---|
| Renin inhibitor | 1 g |
| Sodium chloride | 9 g |
| Methyl p-hydroxybenzoate | 0.5 g |
| Propyl p-hydroxybenzoate | 0.2 g |
| Water for inj. up to | 1000 ml |

The active constituent, sodium chloride and preservatives are dissolved in the main part of the water, whereafter the volume is adjusted to 1000 ml. The solution is filtered through a sterile 0.2 µm filter and aseptically filled into sterile ampoules (5 ml).

Example 49

Solution for nasal administration

A solution is prepared from the following ingredients:

| | |
|---|---|
| Renin inhibitor | 10 g |
| Glycerol | 200 g |
| Methyl p-hydroxybenzoate | 1 g |
| Propyl p-hydroxybenzoate | 0.2 g |
| Water for inj. up to | 1000 ml |

The active constituent and the preservatives were dissolved in glycerol and the main part of the water. The volume is then adjusted to 1000 ml and the solution is filled into sterile polyethylene containers.

Example 50

Tablets for oral administration

1000 tablets are prepared from the following ingredients:

| | |
|---|---|
| Renin inhibitor | 10 g |
| Lactose | 100 g |
| Polyvinyl pyrrolidone | 20 g |
| Avicel | 20 g |
| Magnesium stearate | 2 g |

The active constituent and lactose are mixed with an aqueous solution of polyvinyl pyrrolidone. The mixture is dried and milled to form granules. The Avicel and then the magnesium stearate are then admixed. The mixture is then compressed in a tablet machine giving 1000 tablets, each containing 10 mg of active constituent.

Example 51

Gelatine capsules for oral administration

Gelatin capsules are filled with a mixture of the following ingredients:

| | |
|---|---|
| Renin inhibitor | 10 mg |
| Magnesium stearate | 2 mg |
| Lactose | 188 mg |

Biological data

The potencies of the compounds of the invention as renin inhibitors were determined in vitro using a human renin/angiotensinogen reaction. The assay is based on the method of Ikeda et al (J. Clin. Endocrinal Metab. 54, 423 (1982)), and relies on a radioimmunometric determination of the amount of angiotensin I released from angiotensinogen by renin in a human plasma pool.

The compounds of the invention were dissolved in 0,1 M acetic acid (10 microliter) and then added to human plasma (200 microliter) containing EDTA and 0,6 M citrate buffer (20 microliter). After incubation for 60 minutes at 37° C, $^{125}I$-labelled angiotensin I containing 0,5 mg/ml pepstatine A was added. Antibody coated spheres were added and the resulting mixture incubated for 3 hours at room temperature. 2 ml of distilled water was then added, whereafter the liquid was removed with an aspirator. The radioactivity of the spheres was then determined using a gamma scintillation technique.

The potencies of the compounds thus obtained (see table 1) are given as means of at least four experiments and are expressed as $pIC_{50}$, i.e. the negative logarithm of the molar concentration required to cause 50 % inhibition.

Table 1. Summary of working examples

| Example No | | pIC$_{50}$ |
|---|---|---|
| 1. | Dba-Agl-Cha-[OH]-Ala-[R]-$SO_2$-$CH(CH_3)_2$ | 8.8 |
| 2. | Dnma-Ape-Cha-[OH]-Ala-[R]-$SO_2$-$CH(CH_3)_2$ | 8.2 |
| 3. | Dba-Ape-Cha-[OH]-Ala-[R]-$SO_2$-$CH_3$ | 7.8 |
| 4. | Dnma-Agl-Cha-[OH]-Ala-[R]-$SO_2$-$CH_2CH(CH_3)_2$ | 7.1 |
| 5. | Dba-Agl-Cha-[OH]-Ala-[R]-$SO_2$-cyclohexyl | 8.5 |
| 6. | Dba-Ape-Cha-[OH]-Ala-[R]-$SO_2$-phenyl | 7.9 |
| 7. | Dnma-Agl-Cha-[OH]-Ala-[R]-$SO_2$-$CH_2$-phenyl | 7.5 |
| 8. | Dba-Ape-Cha-[OH]-Ala-[R]-$SO_2$-$CH_2$-cyclohexyl | |
| 9. | Dba(2,3,2',3'-Cl)-Ape-Cha-[OH]-Ala-[R]-$SO_2$-cyclohexyl | 7.4 |
| 10. | Dba(2,3,2',3'-Me)-Agl-Cha-[OH]-Ala-[R]-$SO_2$-cyclohexyl | 7.8 |
| 11. | Dba(4,4'-OH)-Ape-Cha-[OH]-Ala-[R]-$SO_2$-$CH(CH_3)_2$ | 7.4 |
| 12. | Dba-His-Cha-[OH]-Ala-[R]-$SO_2$-$CH(CH_3)_2$ | 6.7 |
| 13. | Dba(4-OH)-Ape-Cha-[OH]-Ala-[R]-$SO_2$-$CH(CH_3)_2$ * | 8.0 |
| 14. | Dba(4,4'-OMe)-Ape-Cha-[OH]-Ala-[R]-$SO_2$-cyclohexyl | 6.6 |
| 15. | Dba(4,4'-OMe)-Agl-Cha-[OH]-Ala-[R]-$SO_2$-$CH_2$-phenyl | 6.2 |
| 16. | Dba(4,4'-$NO_2$)-Ape-Cha-[OH]-Ala-[R]-$SO_2$-$CH_2$-phenyl | 5.9 |
| 17. | Dba(4,4'-$NO_2$)-Agl-Cha-[OH]-Ala-[R]-$SO_2$-cyclohexyl | 6.7 |

| Example No | | pIC$_{50}$ |
|---|---|---|
| 18. | Dba(4,4'-Cl)-Ape-Cha-[OH]-Ala-[R]-$SO_2$-cyclohexyl | 7.1 |
| 19. | Dtma-Cpg-Cha-[OH]-Ala-[R]-$SO_2$-$CH(CH_3)_2$ | 7.4 |
| 20. | Dba-Cpg-Cha-[OH]-Ala-[R]-$SO_2$-$CH(CH_3)_2$ | 7.3 |
| 21. | Dpa-Agl-Cha-[OH]-Ala-[R]-$SO_2$-$CH(CH_2)_2$ | 5.8 |
| 22. | Dpea-Ape-Cha-[OH]-Ala-[R]-$SO_2$-$CH(CH_3)_2$ | 6.2 |
| 23. | Bnma-Val-Cha-[OH]-Ala-[R]-$SO_2$-$CH(CH_3)_2$ * | 7.8 |
| 24. | Dba-Tal-Cha-[OH]-Ala-[R]-$SO_2$-$CH(CH_3)_2$ | 6.6 |
| 25. | Dcma-Ape-Cha-[OH]-Ala-[R]-$SO_2$-$CH(CH_3)_2$ | 6.4 |
| 26. | Dnma-Phg-Cha-[OH]-Ala-[R]-$SO_2$-$CH(CH_3)_2$ | 7.8 |
| 27. | Dba-Ape-Leu-[OH]-Ala-[R]-$SO_2$-$CH(CH_3)_2$ | 6.1 |
| 28. | Dnma-Agl-Leu-[OH]-Ala-[R]-$SO_2$-$CH(CH_3)_2$ | 8.1 |
| 29. | Dba-Ape-Cha-[OH]-Gly-[R]-$SO_2$-$CH(CH_3)_2$ | 7.8 |
| 30. | Dnma-Agl-Cha-[OH]-Gly-[R]-$SO_2$-$CH(CH_3)_2$ | 8.4 |
| 31. | Dba-Agl-Cha-[OH]-Val-[R]-$SO_2$-$CH(CH_3)_2$ | 7.4 |
| 32. | Dnma-Ape-Cha-[OH]-Val-[R]-$SO_2$-$CH(CH_3)_2$ | |
| 33. | Dba-Ape-Cha-[OH]-Ala-[R]-SO-$CH(CH_3)_2$ | |
| 34. | Dba-Agl-Cha-[OH]-Ala-[R]-S-$CH(CH_3)_2$ | |
| 35. | Dbc-Cya-Cha-[OH]-Ala-[R]-$SO_2$-$CH(CH_3)_2$ | 5.0 |

| Example No | | $pIC_{50}$ |
|---|---|---|
| 36. | Bpma-Cpra-Cha-[OH]-Ala-[R]-$SO_2$-$CH(CH_3)_2$ * | 8.6 |
| 37. | Dnma-Ips-Cha-[OH]-Ala-[R]-$SO_2$-$CH(CH_3)_2$ | 7.8 |
| 38. | Bnma-Agl-Cha-[OH]-Ala-[R]-$SO_2$-$CH(CH_3)_2$ * | 8.7 |
| 39. | Bcma-Ape-Cha-[OH]-Ala-[R]-$SO_2$-$CH(CH_3)_2$ | 7.4 |
| 40. | Dpp-Ape-Cha-[OH]-Ala-[R]-$SO_2$-$CH(CH_3)_2$ | |
| 41. | Dba-Fape-Cha-[OH]-Ala-[R]-$SO_2$-$CH(CH_3)_2$ * | |
| 42. | Dba-Cpra-Cha-[OH]-Ala-[R]-$SO_2$-$CH(CH_3)_2$ | 8.3 |
| 43. | Dba-Ape-Cha-[OH]-Ala-[R]-S-$CH(CH_3)_2$ | |
| 44. | Bpma-Agl-Cha-[OH]-Ala-[R]-$SO_2$-$CH(CH_3)_2$ * | |
| 45. | Dtma-Ape-Cha-[OH]-Ala-[R]-$SO_2$-$CH(CH_3)_2$ | 8.3 |
| 46. | Dba-Ala-Cha-[OH]-Ala-[R]-$SO_2$-$CH(CH_3)_2$ | 5.9 |
| 47. | Dnma-Agl-Cha-[OH]-Ala-[R]-$SO_2$-$CH(CH_3)_2$ | 8.5 |

* Diastereomeric mixture

Table 2. NMR data for the compounds of the working examples

Example No 1
$^1$H-NMR ($CDCl_3$): 0.8-1.8 (m,24H)-thereof 1.25 (d,3H), 1.38 (d,3H), 1.40 (d,3H); 2.19 (m,1H); 2.15-2.35 (m,2H); 2.4-3.1 (m,8H); 3.50(m,1H); 3.62 (m,1H); 3.78 (bs,1H); 4.29 (bq,1H); 4.78 (d,1H); 4.88 (d,1H); 5.30 (m,1H); 5.70 (bd,1H); 5.85 (bd,1H); 7.1-7.4 (m,10H).

Example No 2
$^1$H-NMR ($CDCl_3$) :
0.50-1,80(m,32H)-thereof 1.08(d,3H), 1.24(two overlapping d, 6H); 2.00(m,1H); 2.59(dd,1H); 2.85(m,2H); 3.08(m,1H); 3.31-3.60(m,5H); 3.70(bd,1H); 4.15(m,1H); 5.62(bd,1H); 5.87 (bd,1H); 7.20-7.55(m,8H); 7.62(d,1H); 7.69(d,1H); 7.75(d,1H); 7.80(d,1H); 7.85(d,1H); 7.91(d,1H).

Example No 3
$^1$H-NMR ($CDCl_3$) :
0.7-1.8(m,25H)-thereof 0.72(distorted t,3H), 1.15(d,3H); 2.28 (m,1H); 2.70-2.95(m,8H)-thereof 2.90(s,3H); 3.021(dd,1H); 3.12 (dd,1H); 3.52(m,1H); 3.60(m,2H); 4.16(m,1H); 5.75(bd,1H); 5.98 (bd,1H); 7.10-7.35(m,10H).

Example No 4
$^1$H-NMR ($CDCl_3$) :
0.7-1.75 (m,24H)-thereof 1.05 (d,3H), 1.07 (d,3H), 1.09 (d,3H); 1.83 (m,1H); 2.05 (m,2H); 2.25 (m,1H); 2.64 (dd,1H); 2.72 (m,2H); 2.92 (dd,1H); 3.08 (m,1H); 3.3-3.62 (m,7H); 4.15 (q,1H); 4.48 (d,1H); 4.67 (d,1H); 5.1 ( m,1H); 5.42 (d,1H); 5.7 (d,1H); 7.3-7.57 (m,8H); 7.74 (m,3H); 7.85 (m,3H)

Example No 5
$^1$H-NMR ($CDCl_3$) : 0.8-1.8 (m,24H)-thereof 1.16 (d,3H); 2.04 (bd,2H); 2.1-2.4 (m,5H); 2.7-3.1 (m,8H); 3.5-3.65 (m,2H); 3.83 (d,1H); 4.32 (q,1H); 4.78 (d,1H); 4.87 (d,1H); 5.29 (m,1H); 6.00 (t,2H); 7.1-7.4 (m,10H).

Example No 6
$^1$H-NMR ($CDCl_3$) :
0.68-1.80(m,25H)-thereof 0.70(distorted t,3H), 1.15(d,3H); 2.23(m,1H); 2.70-3.06(m,6H); 3.12(dd,1H); 3.47(m,1H);3.58 (m,1H); 3.70(bd,1H), 4.20(m,1H); 5.79(bd,1H); 5.85(d,1H); 7.10-7.35((m,10H); 7.58(t,2H); 7.68(t,1H); 7.90(d,2H).

Example No 7
$^1$H-NMR ($CDCl_3$) :
0.75-1.75(m,18H)-thereof 1.00(d,3H); 1.85(m,2H); 2.08(m,1H); 2.77(dd,1H); 3.10(m,1H); 3.32-3.50(m,7H); 3.56(dd,1H); 4.00 (two AB-d,2H); 4.35(q,1H); 4.48(d,1H); 4.66(d,1H); 5.30(m,1H); 5.48(d,1H); 5.60(d,1H); 7.25-7.48(m,13H); 7.70-7.87(m,6H).

Example No 9
$^1$H-NMR ($CDCl_3$) :
0.71-2.19(m,36H)-thereof 0.73(distorted t,3H); 1.11(d,3H); 2.74-2.88(m,3H); 3.00-3.17(m,6H); 3.39(m,1H); 3.64(m,1H); 4.26(m,1H); 5.72(bd,1H); 6.00(bd,1H); 7.07-7.22(m,4H); 7.32-7.35(two overlapping d,2H).

Example No 10
$^1$H-NMR ($CDCl_3$) :
0.80-1.98(m,28H)-thereof 1.11(d,3H); 2.08-2.28(m,15H)-thereof 2.14(s,3H), 2.16(s,3H), 2.26(s,2H), 2.27(s,3H); 2.57(m,1H); 2.69-3.08(m,7H); 3.43(m,1H); 3.58(m,1H); 4.30(q,1H); 4.75 (dd,1H); 4.87(dd,1H); 5.27-5.38(m,2H); 5.70-5.76(two overlapping d,2H); 6.95-7.05(m,6H).

Example No 11
$^1$H-NMR (DMSO-$d_6$) :
0.70-1.09(m,31H)-thereof 0.78(t,3H), 1.12(d,3H), 1.28(two overlapping d,6H); 2.25(m,1H); 2.55(m,2H); 2.68(m,1H); 2,83 (m,3H); 3.10(dd,1H); 3.23(sept,1H); 3.51(m,1H); 3.81(m,1H); 4.16(m,1H); 4.70(d,1H); 6.64(d,2H); 6.71(d,2H); 6.92(d,2H); 7.03(d,2H); 7.18(d,1H); 7.83(d,1H); 9.10(s,1H); 9.16(s,1H).

Example No 12
$^1$H-NMR ($CDCl_3$) : 0.7-1.8 (m,24H)-thereof 1.15 (d,3H), 1.38 (dd,6H); 2.27 (m,1H); 2.43 (m,1H); 2.7-3.2 (m,10H); 3.39 (bs,1H); 3.50 (m,1H); 4.42 (m,1H); 5.82 (bd,1H); 6.43 (bs,1H); 6.72 (bs,1H); 7.1-7.4 (m,11H); 7.44 (s,1H).

Example No 13
$^1$H-NMR ($CDCl_3$) : Mixture of two diasteromers whereof one gives rise to two rotamers 0.65-1.65 (m,2x31H); 2.25 (m,1H); 2.35 (m.1H); 2.45 (m, minor rotamer); 2.63-3.16 (m,2x8H); 3.45-3.62 (m,2x2H); 3.68 (m,minor

rotamer); 3.38 (m,minor rotamer); 4.07-4.24 (m,2x2H); 5.30 (d,minor rotamer); 5.80 (d,1H); 5.96-6.08 (m,3H); 6.35 (d,minor rotamer); 6.71 (d,2H); 6.75 (d,2H); 6.96 (d,2H); 7.03 (d,2H); 7.10-7.32 (m,2x4H).

Example No 14
$^1$H-NMR ($CDCl_3$) : 0.7-2.2 (m,33H)-thereof 0.74 (t,3H), 1.16 (d,3H); 2.33 (m,1H); 2.6-3.1 (m,8H); 3.55-3.65 (m,2H); 3.75 (s+m,4H); 3.77 (s,3H); 5.90 (bd,1H); 6.08 (bs,1H); 6.77 (d,2H); 6.82 (d,2H); 7.04 (d,2H); 7.13 (d,2H).

Example No 15
$^1$H-NMR ($CDCl_3$) :
0.75-1.75(m,18H)-thereof 1.10(d,3H); 2.10(m,1H); 2.28(m,2H); 2.55-3.00(m,7H); 3.48(m,1H); 3.62(m,1H); 3.71-3.80(m,7H)-thereof 3.75(two s,6H); 4.20-4.30(m,3H)-thereof 4.25(s,2H); 4.82(dd,1H); 4.89(dd,1H); 5.28(m,1H); 5.72(bd,1H); 5.95(bd,1H);6.80(dd,4H); 7.05(d,2H); 7.11(d,2H); 7.40(m,5H).

Example No 16
$^1$H-NMR ($CDCl_3$) :
0.64-1.75(m,25H)-thereof 0.68(distorted t,3H); 1.05(d,3H); 2.25(m,1H); 2.65-2.78(m,5H); 3.06(dd,1H); 3.16(dd,1H); 3.35 (bs,1H); 3.56(m,2H); 4.18-4.30(m,3H)-thereof 4.25(s,2H); 5.80 (d,1H); 6.00(d,1H); 7.28-7.45(m,9H)-thereof 7.31(d,2H),7.36 (d,2H); 8,10-8.17(two overlapping d,4H).

Example No 17
$^1$H-NMR ($CDCl_3$) : 0.8-1.8 (m,24H)-thereof 1.13 (d,3H); 1.95 (bd,2H); 2.1-2.4 (m,5H); 2.7-2.9 (m,5H); 2.96 (dd,1H); 3.10 (dd,1H); 3.18 (dd,1H); 3.49 (d,1H); 3.59 (m,1H); 3.64 (m,1H); 4.30 (q,1H); 4,85 (dd,1H); 4.88 (d,1H); 5.30 (m,1H); 5,82 (d,1H); 6.08 (d,1H); 7.33 (d,2H); 7,38 (d,2H), 8.14 (d,2H); 8.16 (d,2H).

Example No 18
$^1$H-NMR ($CDCl_3$) :
0.70-2.30(m,36H)-thereof 0.76(distorted t,3H), 1.14(d,3H); 2.65(m,1H); 2.70-3.15(m,8H); 3.45(m,1H); 3.65(m,1H); 3.83 (bs,1H); 4.24(m,1H); 5.46(d,1H); 6.01(d,1H); 7.08(d,2H); 7.17(d,2H); 7.21(d,2H); 7.26(d,2H).

Example No 19
$^1$H-NMR ($CDCl_3$) :
0.75-1,80(m,32H)-thereof 1.18(d,3H), 1.39(two overlapping doublets,6H); 2.40(m,1H); 2.80(m,2H); 3.05-3.14(m,4H); 3.21 (m,2H); 3.44(bd,1H); 3.64(m,1H); 3.70(m,1H); 4.17(m,1H); 5.95 (m,2H); 6.80-7.20(m,6H)-thereof 7.12(d,1H); 7.15(d,1H).

Example No 20
$^1$H-NMR ($CDCl_3$) :
0.65-1.80(m,33H)-thereof 1,18(d,3H); 1.39(two overlapping d,6H); 2.05(m,1H); 2.34(m,1H); 2.73-3.15(m,7H); 3.63(m,2H); 3.75(d,1H); 4.15(m,1H); 5.98(bd,1H); 6.01(bd,1H); 7.10-7.35 (m,10H).

Example No 21
$^1$H-NMR ($CDCl_3$) : 0.80 (m,1H); 0.92 (m,1H); 1.05-1.82 (m,22H)-thereof 1.14 (d,3H), 1.35 (dd,6H); 2.39 (m,1H); 2.48 (m,2H); 2.77 (dd,1H); 3.02 (dd,1H); 3.08 (m,1H); 3.13 (m,1H); 3.65 (m,1H); 3.82 (m,1H); 4.48 (Q,1H); 4.94 (s,1H); 4.98 (m,2H); 5.64 (m,1H); 6.22 (m,2H); 7.20-7.38 (m,10H).

Example No 22
$^1$H-NMR ($CDCl_3$) :
0.70-2.05(m,36H);-thereof 0.95(t,3H), 1.18(d,3H), 1.36(d,6H); 2.17(m,1H); 2.35-2.70(m,4H); 2.78(dd,1H); 3.05(m,2H); 3.65 (bd,1H); 3.70(bd,1H); 3.90(m,1H); 4.56(m,1H); 6.32(d,1H); 6.65 (d,1H); 7.05(m,10H).

Example No 23 (mixture of two diastereomers)
$^1$H-NMR ($CDCl_3$) : 0.38 (d); 0.47 (d); 0.51 (d); 0.58 (d); 0.8-1.9 (m)-thereof 1.08 (d), 1.14 (d), 1.31 (d), 1.32 (d), 1.33 (d), 1.34 (d); 2.0 (m); 2.29 (m); 2.61 (dd); 2.75 (dd); 2.85 -3.85 (m); 4.03 (dd); 4.15 (dd); 5.78 (d), 5.80 (d), 5.85 (d); 5.98 (d); 7.15-7.55 (m); 7.65-7.95 (m).

Example No 24
$^1$H-NMR ($CDCl_3$) : 0.79-0.90 (m,2H); 1.07-1.61 (m,22H); 2.12 (m,1H); 2.66-3.12 (m,10H); 3.33 (M,1H); 3.52 (m,1H); 3.65 (m,1H); 4.57 (dd,1H); 5.52 (d,1H); 5.94 (d,1H); 6.38 (d,1H); 6.81 (t,1H); 7.05 (d,1H); 7.12-7.30 (m,10H).

Example No 25
$^1$H-NMR ($CDCl_3$) : 0.76-1.84 (m,57H)-thereof 1.14 (d,3H), 1.35 (d,6H); 2.36-2.50 (m,2H); 2.59 (m,1H); 2.80 (dd,1H); 3.05-3.15 (m,2H); 3.72 (m,1H); 3.87 (m,1H); 4.50 (q,1H); 6.79 (d,1H); 6.87 (d,1H).

Example No 26
$^1$H - NMR ($CDCl_3$) : 0.7-1.8 (m,24H)-thereof 0.94 (d,3H), 1.22 (d,3H), 1.26 /d,3H); 2.08 (m,1H); 2.55 (dd,1H); 2.72 (dd,1H); 2.89 (m,1H); 3.07 (bs,1H); 3.11 (m,1H); 3.29 (dd,1H); 3.33 (dd,1H); 3.41 (dd,1H); 3.50 (m,1H); 3.58 (dd,1H); 3.67 (m,1H); 5.34 (d,1H); 6.12 (d,1H); 6.40 (d,1H); 6.84 (d,2H); 7.0-7.9 (m,17H).

Example No 27
$^1$H-NMR ($CDCl_3$) : 0.67-1.70 (m,27H)-thereof 0.70 (t,3H), 0.88 (d,3H), 0.89 (d,3H), 1.14 (d,3H), 1.38 (d,3H); 1.39 (d,3H); 2.23 (m,1H); 2.70-3.10 (m,8H); 3.41 (m,1H); 3.62 (m,1H); 3.79 (m,1H); 4.21 (m,1H); 5.72 (d,1H); 5.86 (d,1H); 7.12-7.30 (m,10H).

Example No 28
$^1$H-NMR ($CDCl_3$) : 0.86 (d,3H); 0.88 (d,3H); 1.09 (d,3H); 1.26 (d,3H); 1.28 (d,3H); 1.30-1.52 (m,5H); 1.88 (m,1H); 2.00-2.08 (m,2H); 2.60 (dd,1H); 2.84-2.94 (m,2H); 3.04 (m,1H); 3.34-3.49 (m,5H); 3.52-3.59 (m,2H); 4.16 (q,1H); 4.50 (dd,1H); 4.67 (dd,1H); 5.13 (m,1H); 5.50 (d,1H); 5.73 (d,1H); 7.32-7.49 (m,8H); 7.71-7.87 (m,6H).

Example No 29
$^1$H-NMR ($CDCl_3$) : 0.70-1.75 (m,28H)- thereof 0.73 (t,3H), 1.37 (d,3H), 1.38 (d,3H); 1.83-2.00 (m,2H); 2.70 (m,1H); 2.78-3.13 (m,8H); 3.47 (m,1H); 3.68 (m,1H); 4.10 (q,1H); 5.55 (d,1H); 5.87 (d,1H); 7.12-7.32 (m,10H).

Example No 30
$^1$H-NMR ($CDCl_3$) : 0.73-1.70 (m,22H)- thereof 1.35 (d,6H); 1.76-1.95 (m,2H); 2.03 (m,1H); 2.70 (m,1H); 2.82-2.92 (m,2H); 3.03-3.08 (m,2H); 3.32-3.47 (m,4H); 3.58-3.68 (m,2H); 3.97 (q,1H); 4.39 (dd,1H); 4.63 (dd,1H); 5.02 (m,1H); 5.11 (d,1H); 5.68 (d,1H); 7.30-7.53 (m,8H); 7.68-7.94 (m,6H).

Example No 36 The compound consists of two epimers
$^1$H-NMR ($CDCl_3$) :
-0.21- -0.15(m,2x1H); -0.11- -0.02(m,2x1H); 0.09-0.18(m,2x1H), 0.24-0.29(m,2x2H); 0.80-1.75(m,2x27H)-thereof 1.14(two overlapping d,2x3H), 1.39(four overlapping d,2x6H); 2.20(m,1H); 2.26(m,1H); 2.70-2.88(m,2x4H); 2.90-3.12(m,2x4H); 3.42(m,1H); 3.53(m,1H); 3.60-3.67(m,2x1H); 4.26-4.35(m,2x1H); 5.72(d,1H); 5.85(d,1H); 6.17(d,1H); 6.22(d,1H); 7.09-7.33(m,2x7H); 8.42 (broad s,2x1H); 8.51(broad s,2x1H).

Example No 37
$^1$H-NMR ($CDCl_3$) :
0.70-1.85(m,30H) thereof 0.87(d,4H), 0.99(d,3H), 1.13(d,3H), 1.30(dd,6H); 2.31 (m,1H); 2.48(dd,1H); 2.71(q,1H); 2.91-3.11 (m,4H); 3.23-3.37(m,3H); 3.38-3.46(m,2H); 3.50(m,1H); 3.58-3.71(m,2H); 4.13(m,1H); 5.65(d,1H); 5.96(d,1H); 7.29-7.52 (m,8H); 7.67(d,1H); 7.70(d,1H); 7.75(d,1H); 7.80(d,1H); 7.87 (m,1H); 7.93(m,1H).

Example No 42
$^1$H-NMR ($CDCl_3$) : -0.22 (m,1H); -0.09 (m,1H); 0.08 (m,1H); 0.21 (m,1H); 0.23 (m,1H); 0.8-1.3 (m,26H)-thereof 1.14 (d,3H), 1.39 (t,6H); 2.20 (m,1H); 2.7-3.1 (m,8H); 3.40 (m,1H); 3.50 (bs,1H); 3.88 (bd.1H); 4.29 (bq,1H); 5.68 (bd,1H); 5.98 (bd,1H); 7.1-7.4 (m,10H).

Example No 45
$^1$H-NMR ($CDCl_3$) :
0.5-1.9 (m,30H)-thereof 0.79 (t,3H), 1.17 (d,3H), 1.38 (two overlapping d,6H); 2.35 (m,1H); 2.8 (m,2H); 2.95-3.3 (m,6H); 3.55-3.95 (m,3H); 4.3 (m,1H); 6.08 (dd,2H); 6.79-7.17 (m,6H)-thereof 6.82 (d,1H), 6.84-6.95 (m,3H), 7.10-7.16 (t,2H).

## Claims

1. Compounds of the general formula

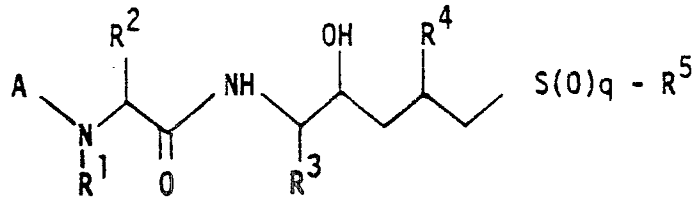

I

where

A = 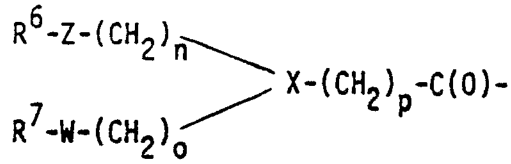.

$R^1$ = H; lower alkyl (1-3 carbons)
$R^2$ = straight or branched lower alkyl or alkenyl (1-6 carbons), optionally substituted with lower alkoxy, cyano, fluoro, lower thioalkyl; cycloalkyl (3-7 carbons); aryl (6-10 carbons); cycloalkylalkyl (4-9 carbons); arylalkyl (7-11 carbons)
$R^3$ = straight or branched lower alkyl (3-6 carbons); cycloalkylalkyl (4-11 carbons); arylalkyl (7-11 carbons)
$R^4$ = H; straight or branched lower alkyl (1-5 carbons)
$R^5$ = straight or branched lower alkyl (1-5 carbons); cycloalkyl (3-7 carbons); aryl (6-10 carbons); arylalkyl (7-11 carbons); cycloalkylalkyl (4-8 carbons)
q = 0-2
o = 0-2
p = 0-2
n = 0-2
X = CH; N
Z = O; $CH(R^8)$; absent
W = O; $CH(R^8)$; absent
$R^6$ = straight or branched lower alkyl (1-6 carbons); cycloalkyl (3-7 carbons); aryl (6-10 carbons) either as such or substituted by 1-3 substituents selected from: halogen (e.g. F,Cl), lower alkoxy (1-3 carbons e.g. methoxy), nitro, hydroxy, lower alkyl (1-3 carbons e.g. methyl), cyano
$R^7$ = as defined for $R^6$
$R^8$ = lower alkyl (1-3 carbons);
however excluding the compound of formula I above wherein A = Dba (i.e. p = 0, X = CH, n = o = l, Z = W = absent, $R^6$ = $R^7$ = phenyl), $R^1$ = H, $R^2$ = propyl, $R^3$ = cyclohexylmethyl, $R^4$ = methyl, q = 2 and $R^5$ = $-CH(CH_3)_2$;
either as such or in the form of a physiologically acceptable salts and including optical isomers.

2. The compound Dba-Agl-Cha-[OH]-Ala-[R]-$SO_2$-$CH(CH_3)_2$
3. The compound Dnma-Ape-Cha-[OH]-Ala-[R]-$SO_2$-$CH(CH_3)_2$
4. The compound Dba-Ape-Cha-[OH]-Ala-[R]-$SO_2$-$CH_3$
5. The compound Dba-Agl-Cha-[OH]-Ala-[R]-$SO_2$-cyclohexyl
6. The compound Dba-Ape-Cha-[OH]-Ala-[R]-$SO_2$-phenyl
7. The compound Dnma-Agl-Cha-[OH]-Ala-[R]-$SO_2$-$CH_2$-phenyl
8. The compound Dba(2,3,2′,3′-Cl)-Ape-Cha-[OH]-Ala-[R]-$SO_2$-cyclohexyl
9. The compound Dba(4,4′-OH)-Ape-Cha-[OH]-Ala-[R]-$SO_2$-$CH(CH_3)_2$
10. The compound Dba(4-OH)-Ape-Cha-[OH]-Ala-[R]-$SO_2$-$CH(CH_3)_2$
11. The compound Dba(4,4′-Cl)-Ape-Cha-[OH]-Ala-[R]-$SO_2$-cyclohexyl
12. The compound Dtma-Cpg-Cha-[OH]-Ala-[R]-$SO_2$-$CH(CH_3)_2$
13. The compound Dba-Cpg-Cha-[OH]-Ala-[R]-$SO_2$-$CH(CH_3)_2$
14. The compound Bnma-Val-Cha-[OH]-Ala-[R]-$SO_2$-$CH(CH_3)_2$
15. The compound Dnma-Phg-Cha-[OH]-Ala-[R]-$SO_2$-$CH(CH_3)_2$
16. The compound Dnma-Agl-Leu-[OH]-Ala-[R]-$SO_2$-$CH(CH_3)_2$
17. The compound Dba-Ape-Cha-[OH]-Gly-[R]-$SO_2$-$CH(CH_3)_2$
18. The compound Dnma-Agl-Cha-[OH]-Gly-[R]-$SO_2$-$CH(CH_3)_2$
19. The compound Dba-Agl-Cha-[OH]-Val-[R]-$SO_2$-$CH(CH_3)_2$
20. The compound Dnma-Ape-Cha-[OH]-Val-[R]-$SO_2$-$CH(CH_3)_2$
21. The compound Bpma-Cpra-Cha-[OH]-Ala-[R]-$SO_2$-$CH(CH_3)_2$
22. The compound Dba-Cpra-Cha-[OH]-Ala-[R]-$SO_2$-$CH(CH_3)_2$
23. The compound Bpma-Agl-Cha-[OH]-Ala-[R]-$SO_2$-$CH(CH_3)_2$
24. A process for preparation of compounds according to any of claims 1-23, wherein an isostere of the formula

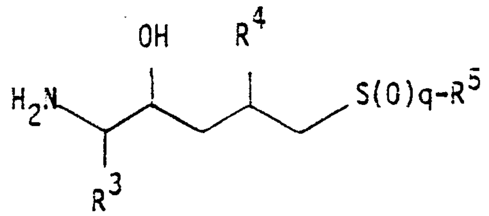

wherein q, $R^3$, $R^4$ and $R^5$ are as defined in any of claims 1-23, is coupled to appropriate amino acids by

standard peptide synthesis techniques, and in those cases where the reactions result in a mixture of diastereomers these are separated by standard chromatographic or recrystallization techniques, and if desired, forming a physiologically acceptable salt and/or isolating an optical isomer.

25. A compound according to any of the claims 1-23 for use as a renin inhibitor.

26. A compound according to any of claims 1-23 for use as a drug for treatment of hypertension, congestive heart failure and other cardiovascular disorders.

27. A pharmaceutical preparation for treatment of hypertension, congestive heart failure and other cardiovascular disorders comprising a therapeutically effective amount of any of the compounds outlined in claims 1-23, and furthermore comprising a pharmaceutical carrier.

28. Use of a compound according to any of claims 1-23 as an active ingredient for manufacture of a pharmaceutical preparation for obtaining inhibition of renin in a human or animal organism.

29. Use of a compound according to any of the claims 1-23 as an active ingredient for manufacture of a pharmaceutical preparation for treatment of hypertension, congestive heart failure and other cardiovascular disorders.

**Claims for the following Contracting States: ES,GR**

1. A process for preparing compounds of the general formula

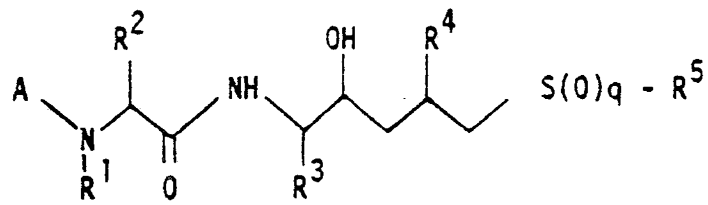

I

where

A =

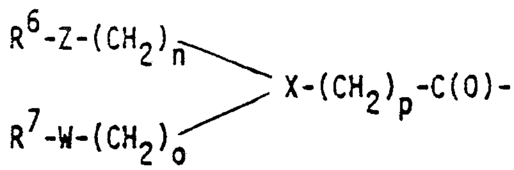

$R^1$ = H; lower alkyl (1-3 carbons)
$R^2$ = straight or branched lower alkyl or alkenyl (1-6 carbons), optionally substituted with lower alkoxy, cyano, fluoro, lower thioalkyl; cycloalkyl (3-7 carbons); aryl (6-10 carbons); cycloalkylalkyl (4-9 carbons); arylalkyl (7-11 carbons)
$R^3$ = straight or branched lower alkyl (3-6 carbons); cycloalkylalkyl (4-11 carbons); arylalkyl (7-11 carbons)
$R^4$ = H; straight or branched lower alkyl (1-5 carbons)
$R^5$ = straight or branched lower alkyl (1-5 carbons); cycloalkyl (3-7 carbons); aryl (6-10 carbons); arylalkyl (7-11 carbons); cycloalkylalkyl (4-8 carbons)
q = 0-2
o = 0-2
p = 0-2
n = 0-2
X = CH; N
Z = O; CH($R^8$); absent
W = O; CH($R^8$); absent
$R^6$ = straight or branched lower alkyl (1-6 carbons); cycloalkyl (3-7 carbons); aryl (6-10 carbons) either as such or substituted by 1-3 substituents selected from: halogen (e.g. F,Cl), lower alkoxy (1-3 carbons e.g. methoxy), nitro, hydroxy, lower alkyl (1-3 carbons e.g. methyl), cyano
$R^7$ = as defined for $R^6$
$R^8$ = lower alkyl (1-3 carbons);
however excluding the compound of formula I above wherein A = Dba (i.e. p = 0, X = CH, n = o = l, Z = W = absent, $R^6$ = $R^7$ = phenyl), $R^1$ = H, $R^2$ = propyl, $R^3$ = cyclohexylmethyl, $R^4$ = methyl, q = 2 and $R^5$ = $-CH(CH_3)_2$;
either as such or in the form of a physiologically acceptable salt and including optical isomers, wherein an isostere of the formula

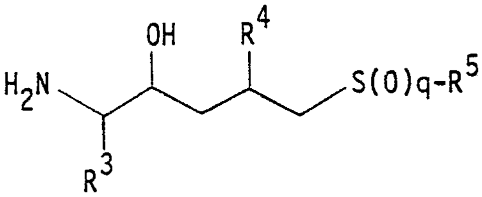

wherein q, $R^3$, $R^4$ and $R^5$ are as defined in any of claims 1-23, is coupled to appropriate amino acids by standard peptide synthesis techniques, and in those cases where the reactions result in a mixture of diastereomers these are separated by standard chromatographic or recrystallization techniques, and if desired, forming a physiologically acceptable salt and/or isolating an optical isomer.

2. A process according to claim 1 for preparing the compound Dba-Agl-Cha-[OH]-Ala-[R]-$SO_2$-$CH(CH_3)_2$

3. A process according to claim 1 for preparing the compound Dnma-Ape-Cha-[OH]-Ala-[R]-$SO_2$-$CH(CH_3)_2$

4. A process according to claim 1 for preparing the compound Dba-Ape-Cha-[OH]-Ala-[R]-$SO_2$-$CH_3$

5. A process according to claim 1 for preparing the compound Dba-Agl-Cha-[OH]-Ala-[R]-$SO_2$-cyclohexyl

6. A process according to claim 1 for preparing the compound Dba-Ape-Cha-[OH]-Ala-[R]-$SO_2$-phenyl

7. A process according to claim 1 for preparing the compound Dnma-Agl-Cha-[OH]-Ala-[R]-$SO_2$-$CH_2$-phenyl

8. A process according to claim 1 for preparing the compound Dba(2,3,2′,3′-Cl)-Ape-Cha-[OH]-Ala-[R]-$SO_2$-cyclohexyl

9. A process according to claim 1 for preparing the compound Dba(4,4′-OH)-Ape-Cha-[OH]-Ala-[R]-$SO_2$-$CH(CH_3)_2$

10. A process according to claim 1 for preparing the compound Dba(4-OH)-Ape-Cha-[OH]-Ala-[R]-$SO_2$-$CH(CH_3)_2$

11. A process according to claim 1 for preparing the compound Dba(4,4′-Cl)-Ape-Cha-[OH]-Ala-[R]-$SO_2$-cyclohexyl

12. A process according to claim 1 for preparing the compound Dtma-Cpg-Cha-[OH]-Ala-[R]-$SO_2$-$CH(CH_3)_2$

13. A process according to claim 1 for preparing the compound Dba-Cpg-Cha-[OH]-Ala-[R]-$SO_2$-$CH(CH_3)_2$

14. A process according to claim 1 for preparing the compound Bnma-Val-Cha-[OH]-Ala-[R]-$SO_2$-$CH(CH_3)_2$

15. A process according to claim 1 for preparing the compound Dnma-Phg-Cha-[OH]-Ala-[R]-$SO_2$-$CH(CH_3)_2$

16. A process according to claim 1 for preparing the compound Dnma-Agl-Leu-[OH]-Ala-[R]-$SO_2$-$CH(CH_3)_2$

17. A process according to claim 1 for preparing the compound Dba-Ape-Cha-[OH]-Gly-[R]-$SO_2$-$CH(CH_3)_2$

18. A process according to claim 1 for preparing the compound Dnma-Agl-Cha-[OH]-Gly-[R]-$SO_2$-$CH(CH_3)_2$

19. A process according to claim 1 for preparing the compound Dba-Agl-Cha-[OH]-Val-[R]-$SO_2$-$CH(CH_3)_2$

20. A process according to claim 1 for preparing the compound Dnma-Ape-Cha-[OH]-Val-[R]-$SO_2$-$CH(CH_3)_2$

21. A process according to claim 1 for preparing the compound Bpma-Cpra-Cha-[OH]-Ala-[R]-$SO_2$-$CH(CH_3)_2$

22. A process according to claim 1 for preparing the compound Dba-Cpra-Cha-[OH]-Ala-[R]-$SO_2$-$CH(CH_3)_2$

23. A process according to claim 1 for preparing the compound Bpma-Agl-Cha-[OH]-Ala-[R]-$SO_2$-$CH(CH_3)_2$

European Patent Office

# EUROPEAN SEARCH REPORT

Application number

EP 89 85 0205.9

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,Y | EP-A-0 172 347 (ABBOT LABORATORIES) *Claims; page 25, exemple 81; page 26, example 86* | 1-29 | C 07 C 323/41<br>C 07 C 317/28<br>C 07 K 5/00 |
| E | EP-A-0 273 893 (AKTIEBOLAGET HÄSSLE) *Whole document* | 1-29 | A 61 K 31/165<br>37/64 |
| Y | WO-A-87/04349 (DELLARIA, JOSEPH et al) *Claim 1* | 1-29 | |
| A | WO-A-88/02374 (THE UPJOHN COMPANY) | 1-29 | |

TECHNICAL FIELDS SEARCHED (Int. Cl.4)

A 61 K

C 07 C

C 07 K

The present search report has been drawn up for all claims

| Place of search | Date of completion of the search | Examiner |
|---|---|---|
| STOCKHOLM | 18-09-1989 | CARLBORG E. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document